(19) Europäisches Patentamt
European Patent Office
Office européen des brevets

(11) Publication number: **0 078 821**
**B1**

(12) # EUROPEAN PATENT SPECIFICATION

(45) Date of publication of patent specification: **28.12.88**

(51) Int. Cl.$^4$: **C 08 G 63/66,** A 01 N 25/30

(21) Application number: **82901424.0**

(22) Date of filing: **12.05.82**

(86) International application number:
**PCT/HU82/00025**

(87) International publication number:
**WO 82/04055 25.11.82 Gazette 82/28**

(54) **TRIGLYCERIDE BASED SURFACTANTS, PROCESS FOR THE PREPARATION THEREOF AND PLANT PROTECTING COMPOSITIONS CONTAINING THE SAME.**

(30) Priority: **13.05.81 HU 132181**

(43) Date of publication of application:
**18.05.83 Bulletin 83/20**

(45) Publication of the grant of the patent:
**28.12.88 Bulletin 88/52**

(84) Designated Contracting States:
**CH DE FR GB LI NL**

(56) References cited:
**DE-A-2 503 768**
**DE-C-2 504 136**
**GB-A-1 459 116**
**GB-A-1 532 506**
**US-A-3 806 479**

**The file contains technical information submitted after the application was filed and not included in this specification**

(73) Proprietor: **CHINOIN Gyogyszer és Vegyészeti Termékek Gyára RT.**
**To utca 1-5**
**H-1045 Budapest IV (HU)**

(72) Inventor: **MOLNAR, Attila**
**6/B Városkuti ut.**
**H-1125 Budapest (HU)**
Inventor: **CSERMELY, György**
**8, Dongó u.**
**H-1149 Budapest (HU)**
Inventor: **RACZ, István**
**26/A, Frakno utca**
**H-1115 Budapest (HU)**
Inventor: **LANYI, György**
**10, Kálló esperes u.**
**H-1124 Budapest (HU)**
Inventor: **MEZES, Ferenc**
**62, Tél utca**
**H-1045 Budapest (HU)**

(74) Representative: **Skailes, Humphrey John et al**
**Frank B. Dehn & Co. Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 078 821 B1

## Description

Technical Field

The present invention relates to triglyceride based surfactants, a process for the preparation thereof and plant protecting compositions comprising the same.

According to an aspect of the present invention there are provided surfactants of the non-ionic and anionic type of the general formula I

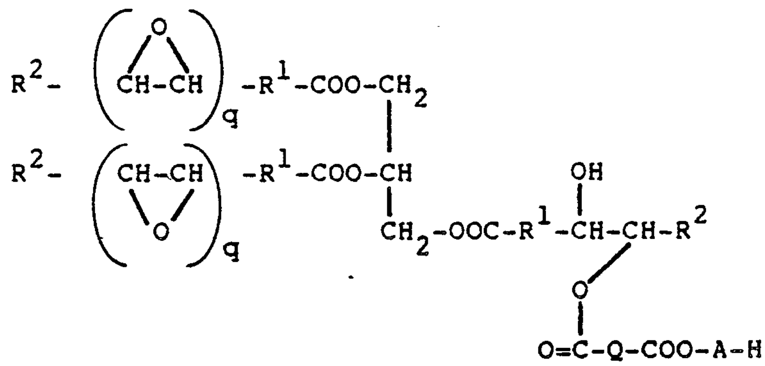

(I)

and/or II

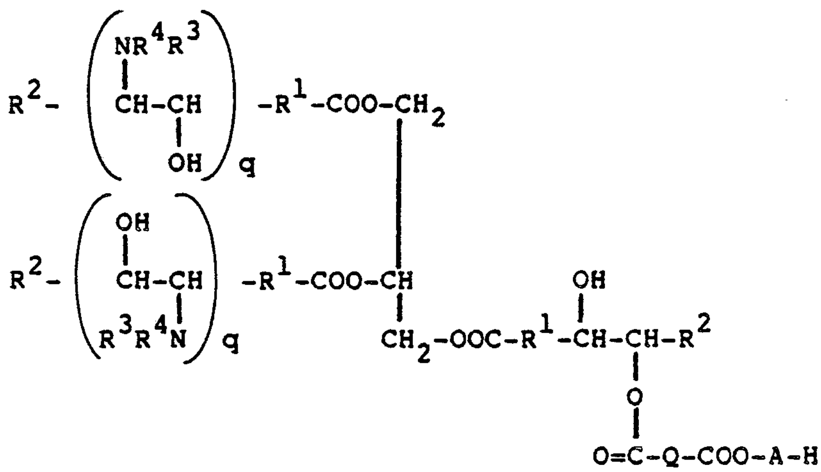

(II)

$R^1$ and $R^2$ together stand for a hydrocarbon chain which when completed by a group of the general Formula $—(CH{=}CH)_q—$ corresponds to the alkyl chain of soya fatty acid,

Q is $—(CH_2)_q—$, $—CH{=}CH—$ or phenylene;

A is $—(CH_2CH_2O)_n—$ or $—[CH(CH_3)CH_2O]_n—$;

$R^3$ and $R^4$ may be the same or different and stand for hydrogen, $C_{1-12}$ alkyl, benzyl, phenyl or alkylaryl;

n is a integer in the range of 3—70;

p is an integer of from 0 to 6;

q is a number of 1.0—1.5.

According to another aspect of the present invention there is provided a process for the preparation of the said compounds of the general Formulae I and II.

According to a still further aspect of the present invention there are provided plant protecting compositions comprising as surface active agent a surfactant of the general Formula I and/or II.

Surfactants of the non-ionic type are mainly alkylene oxide adducts. The alkylene side chain constitutes the hydrophilic part of the molecule while the mostly water-insoluble moiety attached thereto is the hydrophobic part. The most well-known representatives of the said non-ionic surfactants are the ethylene oxide adducts which can be prepared by subjecting reactive ethylene oxide to a polycondensation reaction with a compound containing hydrogen of acidic character.

As compounds containing hydrogen of acidic character alkyl phenols, fatty alcohols, fatty acids, fatty acid amides, amino compounds prepared from fatty acids and mercaptans can be used.

Thus by changing the polar and/or apolar part(s) various types of surfactants can be prepared, and the properties thereof can be varied between wide ranges and modified deliberately.

Background Art

The first basic types of ethoxylated non-ionic surfactants — ethoxylated fatty alcohols and fatty acids — appeared on the market in 1932 as detergents. Later alkyl phenols were used as hydrophobic part of the surfactants. These compounds were widely used in all branches of industry using surfactants due to their very advantageous properties (first of all because they are not sensitive to hard water). In the meantime in certain branches of industry more specific surfactants having higher activity were developed by chemical modification of the basic types.

Thus by using fatty acid amides and alkyl amide oxides as hydrophobic part in the said basic types surfactants having excellent foaming capacity and foam stability were prepared. Sulfuric acid esters of ethoxylated fatty alcohols or alkyl phenols (British patent No. 833.146) and sulfuric acid esters of ethoxy-ethoxylated fatty acid amides possess good foaming capacity. The said surfactants can be used for the preparation of shampoos and foam baths.

In certain cases foaming is an undesired phenomenon. In such instances the desired results can be achieved by proper selection of the structure of both the hydrophobic (US Patent No. 2.859.250) and the hydrophilic parts (US Patent No. 3.022.335).

Similarly surfactants having anti-static effects can be obtained by proper selection of the chemical structure, particularly by increasing the length of the ethoxy chain. According to GFR patent No. 1.184.494 the antistatic effect can be achieved by modifying the chemical structure of the hydrophobic part of ethoxylated alkyllaryl derivatives.

In the formulation of biologically active ingredients — e.g. plant protecting compositions — the main role of the surfactant is to ensure the spontaneous formation of a stable emulsion or suspension having a suitable degree of dispersion. All the other advantageous effects which can be attributed to the presence of the surfactant can be regarded as plus effect.

The surfactant can influence the activity of the plant protecting compositions as well. In the case of herbicides, fungicides and insecticides respectively it depends on the surfactant how the composition moistens the plant, fungi and insects respectively. This enables the preparation of surfactants having the most suitable structure to each composition (P. Bechet: The Emulsifier, Marcel Decker, Inc. 1973, New York).

In the abovementioned article and also in other patent specifications surfactants are described which possess certain plus effects in addition to the typical surface active effect taken in the strict sense of the word. The said plus effects can be mainly achieved by chemical modification of the alkyl, aryl or aralkyl adducts regarded as basic types of the ethoxylated non-ionic suffactants.

It is known that certain plant protecting compositions — particularly thio-phosphoric acid and thio-phosphoric acid derivatives and pyrethrins — decompose in solutions formed with organic solvents with the formation of acidic products. The said decomposition products formed accelerate the de-composition. This involves the risk that on storage the active ingredient might decompose in the solution formed with an organic solvent. In such cases the compositions are usually stabilized by adding acid binding agents or other suitable additives.

O,O-diethyl-0-2-quinoxalinyl-phosphorothioate is a highly effective contact insecticide and acaricide and is put on the market mainly in the form of a liquid emulsion concentrate. According to valid international standards emulsion concentrates must be stable for at least two years. According to GRR patent No. 1.903.228 stability is ensured by adding tertiary amines and/or diprotic apolar solvents; thus dimethyl sulfoxide, N-methyl-pyrrolidone and tetramethyl-urea are admixed in an amount of some per cents to the composition.

DE—2,411,764 relates to the stabilization of O,O-diethyl-O,2-quinoxalinyl-phosphorothionate as well. According to the said patent a compound comprising epoxy group (such as epxoy-cyclohexane, epichlorohydrin or dicyclohexyl-carbodiimide) is added to the solution in an amount of some per cents.

The use of the above stabilizing agents is however accompanied by difficulties. The tertiary amines and above mentioned epoxy compounds are volatile and toxic and therefore the compositions involve serious toxicity risks to the operator. The use of epichlorohydrin is particularly undesired because the carcinogenic effect thereof has been recently made probable. A further drawback is that some of the above additives are very reactive compounds which is a rather undesired property of a stabilizer. Moreover the above additives are rather expensive even when used in a small amount. A further disadvantage is that the said additives might disturb the complicated analysis of the composition comprising a large number of components.

Disclosure of the Invention

It has been found in a surprising manner that the above drawbacks can be eliminated by preparing emulsion concentrates comprising the above mentioned active ingredients and surfactants of the general Formula I and/or II.

The present invention relates to new triglyceride based surfactants of the general Formulae I and II, a process for the preparation thereof and plant protecting compositions comprising the same.

The molecules of the surfactants of the general Formulae I and I contain epoxy and/or amino groups and play a double role in the plant protecting compositions. On the one hand they provide the suitable ready-for-use disperse system and on the other due to the epoxy and amino groups they inhibit the decomposition of the active ingredient in the composition.

In Table I the important properties of some representatives of the compounds of the general Formulae I and II are summarized (q = 1.25).

| No. | A | Q | $R^3$ | $R^4$ | n | Appearance at 25°C | M.p. (°C) | Epoxy-equ. | HLB value |
|---|---|---|---|---|---|---|---|---|---|
| 1 | $-(CH_2CH_2O)_n-$ | $-CH_2CH_2-$ | — | — | 34 | yellowish brown paste | 55—66 | 1000 | 12.5 |
| 2 | $-(CH_2CH_2O)_n-$ | Phenylene | — | — | 34 | brownish paste | 60—65 | 1000 | 12.5 |
| 3 | $-(CH_2CH_2O)_n-$ | $-CH=CH-$ | — | — | 34 | brown paste | 55—60 | 1000 | 12.5 |
| 4 | $-(CH_2CH_2O)_n-$ | $-CH_2CH_2-$ | — | — | 23 | brownish paste | 55—60 | 800 | 11 |
| 5 | $-(CH_2CH_2O)_n-$ | $-CH_2CH_2$ | — | — | 45 | white paste | 60—65 | 1200 | 14 |
| 6 | $-(CH_2CH_2O)_n-$ | $-CH_2CH_2-$ | — | — | 7 | viscous liquid | — | 550 | 5.5 |
| 7 | $-(CH_2CH_2O)_n-$ | $-CH_2CH_2-$ | $n-C_3H_7$ | $n-C_3H_7$ | 34 | brown paste | 55—60 | — | 13.5 |
| 8 | $-(CH_2CH_2O)_n-$ | $-CH_2CH_2$. | $n-C_3H_7$ | $n-C_3H_7$ | 7 | brown paste | 48—51 | — | 7 |
| 9 | $-(CH_2CH_2O)_n-$ | $-CH_2CH_2-$ | $(n-C_4H_9)_2$ | $(n-C_4H_9)_2$ | 34 | brown paste | 50—55 | — | 13.5 |
| 10 | $-[CH(CH_3)CH_2O]_n-$ | $-CH_2CH_2-$ | — | — | 26 | white paste | 60—65 | — | 10.5 |

The compounds of the general Formulae I and II are readily soluble in usual organic solvent and mixtures thereof. Compounds of the general Formulae I and II having a HLB value above 11 give a somewaht opalescent solution in water, while those having a HLB value below 11 form an emulsion. They form moderately foaming aqueous solutions and emulsions respectively.

The compounds of the general Formulae I and II are highly active emulsifiers when emulsifying mineral oil fractions (particularly aromatic fractions) in water. In Table II the decrease of the interfacial water. In Table II the decrease of the interfacial tension between the oily phase and water is shown.

TABLE II
Decrease of interfacial tension when using the surfactant in a concentration of 1 g/l (%)

| Example No. | Sunflower oil/water | Paraffin oil/water | Shellsol[R]/ /water | Xylene/ water |
|---|---|---|---|---|
| 1 | 32.1 | 60.3 | 72.5 | 81.0 |
| 2 | 36.1 | 61.0 | 75.1 | 88.0 |
| 3 | 32.0 | 60.1 | 70.1 | 76.3 |
| 4 | 30.0 | 65.1 | 70.3 | 68.4 |
| 5 | 28.0 | 82.5 | 86.0 | 90.2 |
| 6 | 41.0 | 54.5 | 61.0 | 81.5 |
| 7 | 45.1 | 76.0 | 88.0 | 91.1 |

The non-ionic surfactants of the general Formula I can be easily prepared by two subsequent chemical addition reactions. In the first steo the corresponding polyalkylene glycol and dicarboxylic acid anhydride (e.g. phthalic, maleic, adipic or succinic anhydride) are reacted in the melt or in a suitable solvent to give a half ester. The said half ester can be prepared by methods known per se at 80—120°C, preferably at about 100°C. In the second step the carboxy group of the half ester obtained is reacted with the epoxy group of an epoxidized triglyceride, preferably epoxidized soya oil. The second addition reaction can be carried out at 150—200°C, preferably at 150—170°C. The progress of the reaction can be checked by measuring the acid number.

The advantage of the above process is that no by-products are formed, and both steps can be carried out in the same apparatus by using readily available starting materials.

The anionic type surfactants of the general Formula II can be prepared from the surfactants of the general Formula I by reacting the epoxy groups of the compounds of the general Formula I with suitable amino compounds (e.g. di-n-butyl amine, di-isopropyl-amine) in the presence or absence of a solvent. The reaction can be preferably carried out at 20—80°C.

The surfactants of the general Formulae I and II can be preferably used for the preparation of stable, particularly liquid plant protecting compositions.

Thus in particular the invention provides a liquid composition which comprises 5—70 wt% of thiophosphoric acid, thiophosphoric acid derivatives or pyrethrins as active ingredients) 28 92.5 wt% of one or more carriers and 0—4wt% of the calcium salt of dodecyclobenzene sulfonic acid as auxiliary agent and 1—7.5 wt% of said surfactant.

The surfactants of the present invention are particularly suitable for the stabilisation of emulsion concentrates comprising 25% of O,O-diethyl-O-2-quinoxalinyl-phosphorothioate.

The stability of the compositions of the present invention — emulsion concentrates containing 25% of the active ingredient — is completely equivalent to that of the reference composition comprising epichlorohydrin.

In field experiments the compositions comprising the surfactants of the present invention proved to have biological activity being completely equivalent to that of the reference composition. The compositions were tested in plough-land cultures on beet, potato, tobacco, lucerne, pea, maize, sugar beet, autumn wheat, vegetables and fruits (e.g. grape, apple, peach, plum and cherry) against various pests. In various stages of the cultures excellent biological effect was found but no phitotoxicity was observed.

Best mode of carrying out the invention

Further details of the present invention are to be found in the Examples without limiting the scope of protection to the Examples.

Example 1

50 g of succinic anhydride and 750 g of polyethylene glycol (OH number 75 mg KOH/g) are reacted at 90°C. To the half ester thus obtained 452 g of epoxidized soya oil (epoxy equivalent 250, iodine number 6) are added and the mixture is reacted at 170—180°C until the acid number becomes smaller than 1 mg KOH/g. On cooling a compound of the general Formula I is obtained wherein A is —$(CH_2CH_2O)_{34}$—; Q is —$CH_2CH_2$— and q = 1.25.
Characteristic data: mp.: 55—60°C, OH number 0.05 mg KOH/g, epoxy equivalent 1000, HLB value 12.5.

Example 2

74 g of phthalic anhydride and 750 g of polyethylene glycol (OH number 75 mg KOH) are reacted at 90°C. To the half ester thus obtained 452 g of epoxidized soya oil are added (epoxy equivalent 250, iodine number 6) and the mixture is reacted at 170—180°C until the acid number becomes smaller than 1 mg/KOH/g. On cooling a surfactant of the general Formula I is obtained wherein A is —$(CH_2CH_2O)_{34}$—; Q is phenylene and q is 1.25.
Characteristic data: mp.: 60—65°C, OH number 0.05 mg KOH/g, epoxy equivalent 1000, HLB value 12.5.

Example 3

50 g of succinic anhydride and 500 g of polyethylene glycol (OH number 112 mg KOH/g) are reacted at 90°C. To the half ester thus obtained 452 g of epoxidized soya oil are added (epoxy equivalent 250, iodine number 6) and the mixture is reacted at 170—180°C until the acid number becomes lower than 1 mg/KOH/g. On cooling a surfactant of the general Formula I is obtained wherein A is —$(CH_2CH_2O)_{23}$—; Q = —$CH_2CH_2$— and q = 1.25.
Characteristic data: mp.: 55—60°C, OH number 0.06 mg KOH/g, epoxy equivalent 800, HLB value 11.0.

Example 4

50 g of succinic anhydride and 1000 g of polyethylene glycol (OH number 56 mg KOH/g) are reacted at 90°C. To the half ester thus obtained 452 g of epoxidized soya oil are added (epoxy equivalent 250, iodine number 6) and the mixture is reacted at 170—180°C until the acid number becomes smaller than 1 mg/KOH/g. On cooling a surfactant of the general Formula I is obtained wherein A is —$(CH_2CH_2O)_{34}$—; Q is phenylene and q is 1.25.
Characteristic data: mp.: 60—65°C, OH number 0.04 mg KOH/g, epoxy equivalent 1200, HLB value 14.0.

Example 5

50 g of succinic anhydride are reacted with 150 g of polyethylene glycol (OH number 374 mg KOH/g) at 90°C. To the half ester thus obtained 452 g of epoxidized soya oil are added (epoxy equivalent 250, iodine number 6) and the mixture is reacted at 170—180°C until the acid number becomes lower than 1 mg/KOH/g. On cooling a surfactant of the general Formula I is obtained wherein A is —$(CH_2CH_2O)_7$—; Q is —$CH_2CH_2$—; q = 1.25.
Characteristic data: OH number 0.1 mg KOH/q, epoxy equivalent 520, HLB value 5.5.

Example 6

250 g of a compound of the general Formula I (wherein —$(CH_2CH_2O)_{34}$— and Q = —$CH_2CH_2$—) and 20.2 g of di-*n*-propyl-amine are reacted at 60°C for 90 minutes. On cooling a compound of the general Formula II is obtained wherein A is —$(CH_2CH_2O)_{34}$—; Q is —$CH_2$—$CH_2$—, q = 1.25; $R^3$ and $R^4$ = *n*-propyl.
Characteristic data: OH number 0.1 mg KOH/q, HLB value 13.6.

Example 7

130 g of a compound of the general Formula I (wherein A is —$(CH_2CH_2O)_7$—, Q is —$CH_2$—$CH_2$—) are reacted with 20.2 g of di-*n*-propyl amine at 60°C for 90 minutes. On cooling a compound of the general Formula II is obtained wherein A is —$(CH_2CH_2O)_7$—; Q is —$CH_2$—$CH_2$—; q is 1.25 and $R^3$ and $R^4$ are n-propyl.
Characteristic data: OH number 0.2 mg KOH/q, HLB value 7.

Example 8 (reference Example)

An emulsifiable concentrate (EC) containing 25% of O,O-diethyl-O,2-quinoxalinyl-phosphorothioate is prepared by admixing the following components:

| Component | Amount, parts by weight |
|---|---|
| O,O-diethyl-O-2-quinoxalinylphosphorothioate | 25.0 |
| Nonylphenol-polyglycolether, HLB=12.0 | 3.0 |
| Calcium salt of dodecyl benzene sulfonic acid | 1.0 |
| Xylene | 25.0 |
| Shellsol[R] | 45.0 |
| Epichlorohydrin | 1.0 |

Example 9

| Component | Amount, parts by weight |
|---|---|
| O,O-diethyl-O-2-quinoxalinylphosphorothioate | 25.0 |
| Surfactant of the general Formula I wherein A is $-(CH_2CH_2O)_{34}-$, Q is $-CH_2-CH_2-$, q = 1.25 | 2.8 |
| Calcium salt of dodecyl benzene sulfonic acid | 1.0 |
| Xylene | 71.2 |

Example 10

| Component | Amount, parts by weight |
|---|---|
| O,O-diethyl-O-2-quinoxalinylphosphorothioate | 25.0 |
| Surfactant of the general Formula I [A is $-(CH_2CH_2O)_{34}-$, Q = $-CH{=}CH-$, q = 1.25] | 3.2 |
| Calcium salt of dodecyl benzene sulfonic acid | 1.0 |
| Xylene | 25.0 |
| Shellsol$^R$ | 45.8 |

Example 11

| Component | Amount, parts by weight |
|---|---|
| O,O-diethyl-O-2-quinoxalinylphosphorothioate | 55.0 |
| Surfactant of the general Formula I [A is $-(CH_2CH_2O)_{45}-$, Q is $-CH_2CH_2-$ q = 1.25] | 5.5 |
| Surfactant of the general Formula I [A is $-(CH_2CH_3O)_7-$, Q is $-CH_2-CH_2-$, q = 1.25] | 2.0 |
| Calcium salt of dodecyl benzene sulfonic acid | 2.0 |
| Xylene | 35.5 |

Example 12

| Component | Amount, parts by weight |
|---|---|
| O,O-diethyl-O-2-quinoxalinylphosphorothioate | 5.0 |
| Surfactant of the general Formula II [A is $-(CH_2CH_2O)_{34}-$, Q is $-CH_2-CH_2-$, q = 1.25, $R^3 = R^4 = n$ propyl] | 2.0 |
| Calcium salt of dodecyl benzene sulfonic acid | 0.5 |
| Xylene | 40.0 |
| Isopropanol | 12.5 |
| Shellsol$^R$ | 40.0 |

Example 13

In this Example the application of the compositions according to Examples 8—12 is described and the testing of the activity thereof is disclosed.

The emulsion concentrates according to Examples 8—12 are subjected to the following tests:

Emulsion stability: emulsions of a concentration of 5 vol.% are prepared by using standard hard water (about 19 IH°) and standard soft water (about 2 IH°). The emulsions are allowed to stand at a temperature of 30 ± 1°C and the amount of precipitate is weighed after 2, 24, and 48 hours respectively.

Accelerated testing of the stability of the emulsion concentrate: the samples (20 ml) are stored for 30 minutes under airtight sealing at 40°C, 50°C and 60°C respectively. In the heat-treated samples the amount of the decomposition product (2-hydroxyquinoxaline) is measured by UV spectrophotometric methods.

Thin layer chromatography:
the O.O-diethyl-O-2-quinoxalinyl-phosphorothioate active ingredient content is determined with the aid of a Video-densitometer.

Cold resistance of the emulsion concentrate: the samples (100 ml) are stored at −5°C for 72 hours. Samples which remain as clear solution after this period of time are considered to have adequate cold resistance.

The results are summarised in Table III.

TABLE III

| Sample No. | Emulsion stability; amount of precipitate (g) after | | | Testing of stability; decomposed active ingredient (%) | | | Cold resistance |
|---|---|---|---|---|---|---|---|
| | 2 | 24 hours | 48 | 40°C | 50°C | 60°C | |
| Example 8 (reference) | — | 0.5 | 1.0 | 0.5 | 0.8 | 1.4 | correspond |
| Example 9 | — | — | 0.1 | 0.4 | 0.5 | 0.8 | " |
| Example 10 | — | — | 0.1 | 0.2 | 0.5 | 0.7 | " |
| Example 11 | — | — | 0.1 | — | 0.4 | 0.5 | " |
| Example 12 | — | — | — | — | 0.5 | 0.7 | " |

Example 14

Field tests are carried out by using the emulsion concentrates prepared according to Examples 8—10. The samples are tested against various pests. In order to show the efficiency of the compositions according to the present invention the results of the tests carried out against two pests are given below.

a) Protection against apple ermel (Laspeyresia ponomella L).

Parcel size: 2.5 ha
Sort: Starking
Golden delicious
Replicate (number of tests): 4
Amount of water: 800 l/ha
Samples: Control (untreated area)
EC according to Example 8 (reference sample)
EC according to Example 9 and 10
Concentration: 0.15%

The results obtained are summarised in Table IV.

TABLE IV

| Sample | Effect related to control (%) |
|---|---|
| Reference Example 8 | 81.5 |
| Example 9 | 82.1 |
| Example 10 | 80.5 |

b) Protection against Argyresthia conjugella.

The test is carried out in area strongly infected first of all by Lithocolletis Blancardella Forr and to a smaller extent by Nepticula malella.

Parcel size: 1.3 ha

Replicate number: 2

Sample: control (untreated area)
Reference Example 8
Examples 9 and 10.

Amount of water: 1500 1/ha

Concentration: 0.2%.

The results are summarised in Table V.

TABLE V

| Sample | Effect related to control (%) |
|---|---|
| Reference Example 8 | 80.2 |
| Example 9 | 82.1 |
| Example 10 | 80.6 |

Example 15

| Component | Amount, parts by weight |
|---|---|
| O,O-Dimethyl-(1-hydroxy-2,2,2-trichloro-ethyl)-phosphonate (Trichlorphon) | 70 |
| Surfactant of the general Formula I (wherein A is $-(CH_2CH_2O)_{45}-$, Q is $-CH=CH$ and $q = 1.25$) | 2 |
| 1,2-Propylene-glycol | 28 |

Example 16

| Component | Amount, parts by weight |
|---|---|
| Trichlorphon | 50.0 |
| Xylene | 39.5 |
| 1,2-Propylene-glycol | 8.0 |
| Surfactant of the general Formula I (wherein A is $-(CH_2CH_2O)_{34}-$, $Q = -CH_2-CH_2-$ and $q = 1.25$) | 2.0 |
| Calcium salt of dodecyl benzene sulfonic acid | 0.5 |

Example 17

The tests described in Example 13 are carried out by using the compositions prepared according to Examples 15 and 16. As standard Dipterex 50 is used in the same active ingredient concentration.

According to the application tests the compositions according to Examples 15 and 16 proved to be equivalent to the standard.

The activity tests are carried out under laboratory conditions. The activity of the compositions according to Example 15 and 16 were equivalent to that of the standard composition.

Example 18

| Component | Amount, parts by weight |
|---|---|
| Dichlorovos (O,O-dimethyl-2,2-dichloro-vinyl)-phosphate) | 50.0 |
| Xylene | 39.0 |
| Propyleneglycol-1,2 | 4.5 |
| Surfactant of the general formula I [A is $—(CH_2CH_2O)_{34}—$ Q is phenylene, q = 1.25] | 4.5 |
| Calcium salt of dodecyl benzene sulfonic acid | 2.0 |

The application tests were carried out according to Examples 13. As standard *Vapona 48 EC was used. The composition of the present invention according to Example 18 proved to be equivalent to the standard composition. (*a product of the Shell Chemical Company containing Dichlorvos).

Example 19

| Component | Amount, parts by weight |
|---|---|
| Permethrin [3-phenoxybenzyl-3-(2,2-dichloro-vinyl)-2,2-dimethyl-cyclopropane carboxylic acid] | 23.0 |
| Tetramethrin (3,4,5-tetrahydrophthalimido-methyl-chrysanthemate) | 2.0 |
| Benzyl alcohol | 5.0 |
| Butyl-hydroxy-toluene | 0.1 |
| Propylene-glycol-1,2 | 1.0 |
| Surfactant of the general Formula I (A= $—(CH_2CH_2O)_{23}—$, Q=$—CH_2CH_2—$, q=1.25] | 2.0 |
| Surfactant of the general Formula I [wherein A is $—(CH_2CH_2O)_7—$, Q is $—CH_2—CH_2—$, q = 1.25] | 3.5 |
| Calcium salt of dodecyl benzene sulfonic acid | 4.0 |
| Xylene | 10.0 |
| Sunflower oil | 38.6 |

Example 20

| Component | Amount, parts by weight |
|---|---|
| Permethrin | 23.0 |
| Tetramethrin | 2.0 |
| Benzyl alcohol | 5.0 |
| Butyl-hydroxy-toluene | 0.1 |

Example 20

| Component | Amount, parts by weight |
|---|---|
| 1,2-Propyleneglycol | 5.5 |
| Surfactant of the general Formula I [wherein A is $—(CH_2CH_2O)_{23}—$, Q is —CH=CH— and q = 1.25) | 5.5 |
| Calcium salt of dodecyl benzene sulfonic acid | 4.0 |
| Xylene | 4.9 |
| Shellsol[R] | 50.0 |

Example 21

The application tests of the compositions prepared according to Examples 19 and 20 were carried out as described in Example 13. As standard Ambush 25 EC was used. The composition according to Examples 19 and 20 proved to be equivalent to the standard sample.

**Claims**

1. A plant protecting composition comprising as a surface active agent a suractant of the general Formula I

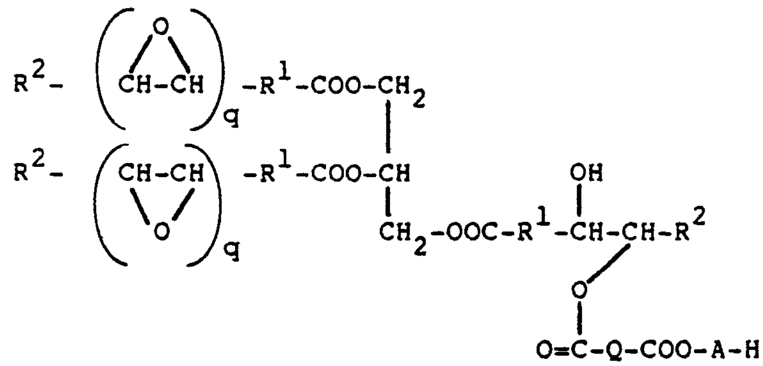

(I)

and/or II

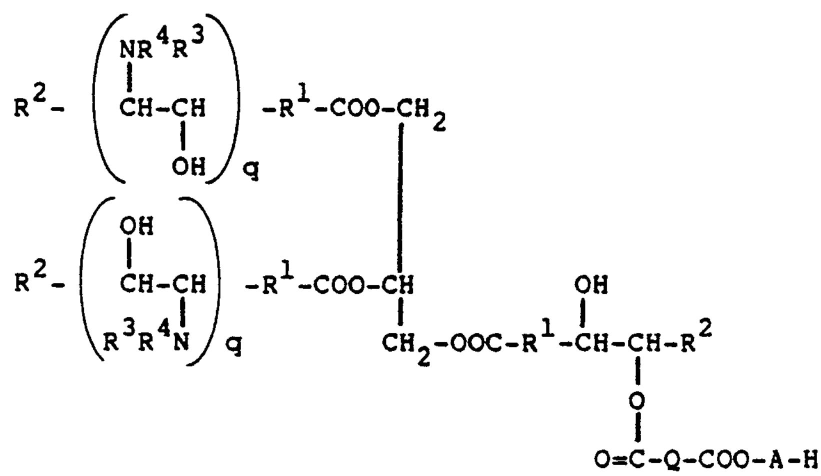

(II)

(wherein

$R^1$ and $R^2$ together stand for a hydrocarbon chain which when completed by a group of the general Formula $—(CH=CH)_q—$ corresponds to the alkyl chain of soya fatty acid;

Q is $—(CH_2)_p—$, —CH=CH— or phenylene;

A is $—(CH_2CH_2O)_n—$ or $—[CH(CH_3)CH_2O]_n—$;

$R^3$ and $R^4$ may be the same or different and stand for hydrogen, $C_{1-12}$ alkyl, benzyl, phenyl or alkylaryl;

n is a integer in the range of 3—70;
p is an integer of from 0 to 6;
q is a number of 1.0—1.5).

2. Composition according to claim 1, comprising O,O-diethyl-O-(2-quinoxalinyl)-phosphorothioate as active ingredient.

3. Composition according to claim 1 comprising O,O-diethyl-(1-hydroxy-2,2,2-trichloromethyl)-phosphonate as active ingredient.

4. Composition according to claim 1 comprising O,O-diethyl-2,2-dichloro-vinyl-phosphate as active ingredient.

5. Composition according to claim 1 comprising 3-phenoxy-benzyl-3-(2,2-dichloro-vinyl)-2,2-dimethylcyclopropane carboxylic acid and/or 3,4,5-tetrahydrophthalimido-methyl-chrysanthenate as active ingredient.

6. Composition according to claim 1 in the form of an emulsion comprising 25% by weight of O,O-diethyl-O-(2-quinoxalinyl)phosphorothioate.

7. Composition according to claim 1 comprising as surfactant a compound of the general Formula I wherein
A is $(CH_2CH_2O)_{34}$—,
Q is —$CH_2$—$CH_2$— and
q is 1.25.

8. Composition according to claim 1 comprising as surfactant a compound of the general Formula I, wherein
A is —$(CH_2CH_2O)_{23}$—;
Q is —$CH_2$—$CH_2$—;
q is 1.25.

9. Composition according to claim 1 comprising as surfactant a compound of the general Formula I wherein
A is —$(CH_2CH_2O)_7$—;
Q is —$CH_2$—$CH_2$— and
q is 1.25.

10. Composition according to claim 1 comprising as surfactant a compound of the general Formula II wherein
A is —$(CH_2CH_2O)_{34}$—;
Q is —$CH_2$—$CH_2$— and
q is 1.25.

11. Composition according to claim 1 comprising as surfactant a compound of the general Formula II wherein
A is —$(CH_2CH_2O)_7$—;
Q is —$CH_2$—$CH_2$— and
q is 1.25.

12. A liquid composition according to any of Claims 1 to 5 and 7 to 11 which comprises:

(i) 5—70 wt% of thiophosphoric acid, thiophosphoric acid derivatives or pyrethrins as active ingredients;

(ii) 28—92.5 wt% of one or more carriers and 0—4 wt% of the calcium salt of dodecylbenzene sulfonic acid as auxiliary agent; and

(iii) 1.75 wt% of said surfactant.

13. A process for the preparation of a surfactant of formula (I) or (II) as defined in claim 1 which comprises:

a) for the preparation of compounds of the general Formula I, reacting epoxidized soya oil with a half ester of a dicarboxylic acid formed with a polyalkylene glycol having a molecular weight of from 200 to 3000 in the presence or absence of a solvent at a temperature of 150—200°C whereby a compound of formula (I) is produced; or

b) for the preparation of compounds of the general Formula II, reacting a compound of the general Formula I (wherein $R^1$, $R^2$, Q, A, n p and q are as stated in Claim 1) with ammonia or a primary or secondary amino compound in the presence or absence of a solvent.

14. Process according to method a) of Claim 13 which comprises using maleic acid, phthalic acid, adipic acid or succinic acid as dicarboxylic acid.

15. Surfactants of the general Formula I as defined in Claim 1.

16. Surfactants of the general Formula II as defined in Claim 1.

**Patentansprüche**

1. Pflanzenschutzkomposition, dadurch gekennzeichnet, daß sie als oberflächenktives Mittel ein Surfactant der allgemeinen Formel I

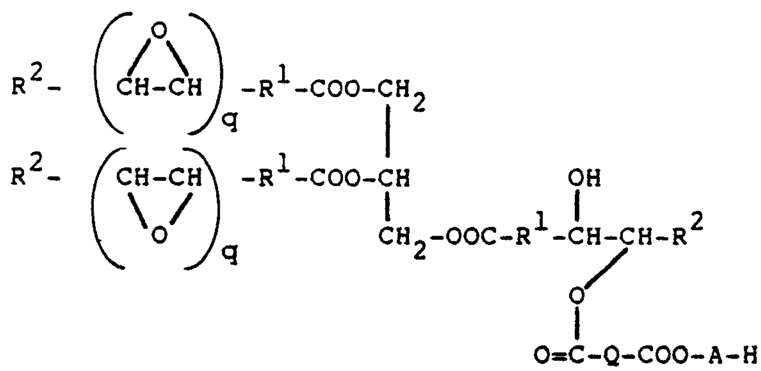

(I)

und/oder II

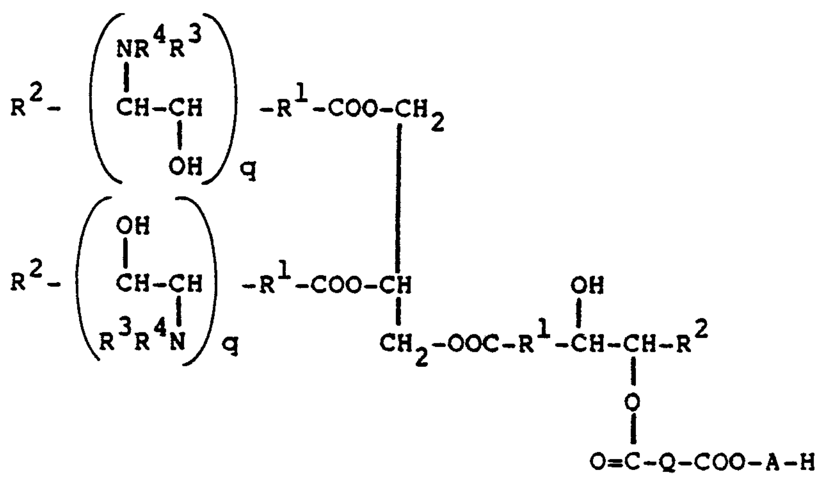

(II)

worin

$R^1$ und $R^2$ gemeinsam eine Kohlenwasserstoffkette bedeuten, die wenn sie durch eine Gruppe der allgemeinen Formel $—(CH{=}CH)_q—$ vervollständigt ist — der Alkylkette der Sojafettsäure entspricht,

Q $—(CH_2)_q—$, $—CH{=}CH—$ oder Phenylen,

A $—(CH_2CH_2O)_n—$ oder $[CH(CH_3)CH_2O]_n—$,

$R^3$ und $R^4$, die gleich oder verschieden sein können, Wasserstoff, $C_{1-12}$-Alkyl, Benzyl, Phenyl oder Alkaryl,

n eine ganze Zahl im bereich von 3 bis 70,

p eine ganze Zahl von 0 bis 6 und

q eine Zahl von 1,0 bis 1,5 bedeuten.

2. Komposition nach Anspruch 1, dadurch gekennzeichnet, daß sie als aktives Ingrediens O,O-Diäthyl-O-(2-chinoxalinyl)-phosphorthioat enthält.

3. Komposition nach Anspruch 1, dadurch gekennzeichnet, daß sie als aktives Ingrediens O,O-Dimethyl-(1-hydroxy-2,2,2-trichloräthyl)-phosphonat enthält.

4. Komposition nach Anspruch 1, dadurch gekennzeichnet, daß sie als aktives Ingrediens O,O-Dimethyl-2,2-dichlor-vinyl-phosphat enthält.

5. Komposition nach Anspruch 1, dadurch gekennzeichnet, daß sie als aktives Ingrediens 3-Phenoxy-benzyl-3-(2,2-dichlor-vinyl)-2,2-dimethyl-cyclopropancarbonsäure und/oder 3,4,5-Tetrahydropthalimidomethyl-chrysanthemat enthält.

6. Komposition nach Anspruch 1 in Form einer Emulsion, gekennzeichnet durch einen Gehalt von 25 Gew.% O,O-Diäthyl-O-(2-chinoxalinyl)-phosphorthioat.

7. Komposition nach Anspruch 1, dadurch gekennzeichnet, daß sie als Surctant eine Verbindung der allgemeinen Formel I enthält,

worin

A $—(CH_2CH_2O)_{34}—$,

Q $—CH_2—CH_2—$ und

q 1,25 bedeuten.

8. Komposition nach Anspruch 1, dadurch gekennzeichnet, daß sie als Surfactant eine Verbindung der allgemeinen Formel I enthält,
worin
A $-(CH_2CH_2O)_{23}-$,
Q $-CH_2-CH_2-$ und
q 1,25 bedeuten.

9. Komposition nach Anspruch 1, dadurch gekennzeichnet, daß sie als Surfactant eine Verbindung der allgemeinen Formel I enthält,
worin
A $-(CH_2CH_2O)_7-$,
Q $-CH_2-CH_2-$ und
q 1,25 bedeuten.

10. Komposition nach Anspruch 1, dadurch gekennzeichnet, daß sie als Surfactant eine Verbindung der allgemeinen Formel II enthält,
worin
A $-(CH_2CH_2O)_{34}-$,
Q $-CH_2-CH_2-$ und
q 1,25 bedeuten.

11. Komposition nach Anspruch 1, dadurch gekennzeichnet, daß sie als Surfactant eine Verbindung der allgemeinen Formel II enthält,
worin
A $-(CH_2CH_2O)_7-$,
Q $-CH_2-CH_2-$ und
q 1,25 bedeuten.

12. Flüssige Komposition nach einem der Ansprüche 1 bis 5 oder 7 bis 11, dadurch gekennzeichnet, daß sie
i) 5 bis 70 Gew.% Thiophosphorsäure, Thiophosphorsäure-Derivate oder Pyrethrine als aktive Ingredientien,
ii) 28 bis 92,5 Gew.% eines oder mehrerer Trägermaterialien und 0 bis 4 Gew.% des Calciumsalzes der Dodecylbenzolsulfonsäure als Hilfsmittel und
iii) 1 bis 7,5 Gew.% Surfactants enthält.

13. Verfahren zur Herstellung eines oberflächenaktiven Mittels der in Anspruch 1 angegebenen Formeln I und/oder II, dadurch gekennzeichnet, daß man
a) zur Herstellung der Verbindungen der allgemeinen Formel I epoxidiertes Sojaöl mit einem Halbester aus einer Dicarbonsäure und einem Polyalkylenglykol mit einem Molekulargewicht von 200 bis 3000 in An- oder Abwesenheit eines Lösungsmittels bei einer Temperatur von 150 bis 200°C umsetzt, wobei eine Verbindung der allgemeinen Formel I gebildet wird, oder
b) zur Herstellung der Verbindungen der allgemeinen Formel II, eine Verbindung der allgemeinen Formel I (worin $R^1$, $R^2$ Q. A, n, p und q die in Anspruch 1 angegebene Bedeutung haben) mit Ammoniak, einem primären oder einem sekundären %Amin in An- oder Abwesenheit eines Lösungsmittels umsetzt.

14. Verfahren nach Anspruch 13, Methode a), dadurch gekennzeichnet, daß man als Dicarbonsäure Malein-, Phthal-, Adipin- oder Bernsteinsäure verwendet.

15. Oberflächenaktive Mittel der in Anspruch 1 angegebenen Allgemeinen Formel I.

16. Oberflächenaktive Mittel der in Anspruch 1 angegebenen allgemeinen Formel II.

**Revendications**

1. Composition protectrice de plantes comprenant comme agent tensionactif un surfactant de formule generale I

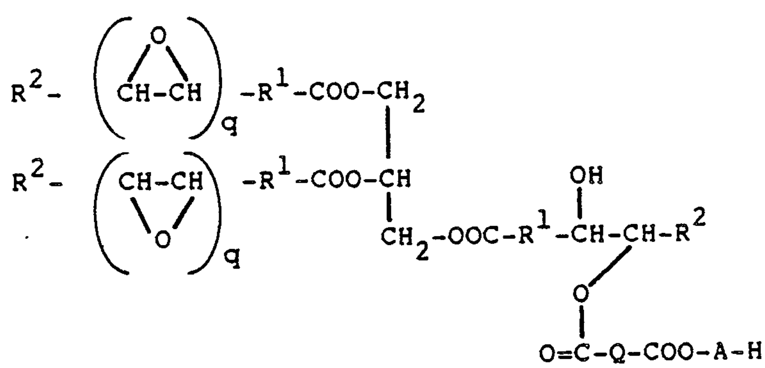

(I)

et/ou II

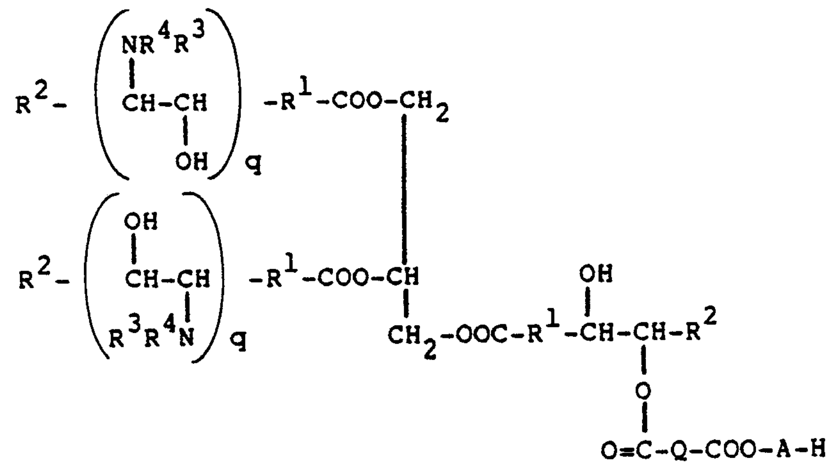

(II)

(dans laquelle

$R^1$ et $R^2$ représentent tous les deux une chaîne hydrocarbonée qui, quand elle est complétée par un groupe de formule générale $—(CH{=}CH)_q—$ correspond à la chaîne alkyle de l'acide gras de soja;

Q est $—(CH_2)_q—$, $—CH{=}CH—$ ou le phénylène;

A est $—(CH_2CH_2O)_n—$ ou $—[CH(CH_3)CH_2O]_n—$;

$R^3$ et $R^4$ peuvent être indentiques ou différents ou représenter l'hydrogène, un alkyle $C_{1-12}$, le benzyle, le phényle ou un alkylaryl;

n est un entier compris entre 3 et 70;

p est un entier compris entre 0 et 6;

q est un nombre compris entre 1,0 et 1,5).

2. Composition selon la revendication 1, comprenant de 1'O,O-diéthyl-O-(2-quinoxalinyl)-phosphorothioate comme ingredient actif.

3. Composition selon la revendication 1, comprenant de 1'O,O-diéthyl-(1-hydroxy-2,2,2-trichlorométhyl)phosphonate comme ingrédient actif.

4. Composition selon la revendication 1, comprenant de 1'O,O-diéthyl-2,2-dichlorovinyl-phosphate comme ingrédient actif.

5. Composition selon la revendication 1, compenant de l'acide 3-phénoxy-benzyl-3-(2,2-dichlorovinyl)-2,2-diméthylcyclopropane-carboxylique et/ou du 3,4,5-tétrahydrophtalimindométhylchrysanthénate comme ingrédient actif.

6. Composition selon la revendication 1, sous la forme d'une émulsion comprenant 25% en poids de O,O-diéthyl-O-(2-quinoxalinyl)-phosphorothioate.

7. Composition selon la revendication 1, comprenant comme surfactant un composé de formule générale I dans laquelle

A est $—(CH_2CH_2O)_{34}—$,

Q est $—CH_2—CH_2—$ et

q est 1,25.

8. Composition selon la revendication 1, comprenant comme surfactant un composé de formule générale I, dans laquelle

A est $—(CH_2CH_2O)_{23}—$;

Q est $—CH_2—CH_2—$;

q est 1,25.

9. Composition selon la revendication 1, comprenant comme surfactant un composé de formule générale I, dans laquelle

A est $—(CH_2CH_2O)_7—$;

Q est $—CH_2—CH_2—$ et

q est 1,25.

10. Composition selon la revendication 1, comprenant comme surfactant un composé de formule générale I, dans laquelle

A est $—(CH_2CH_2O)_{34}—$;

Q est $—CH_2—CH_2—$ et

q est 1,25.

11. Composition selon la revendication 1, comprenant comme surfactant un composé de formule générale I, dans laquelle

A est $—(CH_2CH_2O)_7—$;

Q est $—CH_2—CH_2—$ et

q est 1,25.

12. Composition liquid selon l'une quelconque des revendications 1 à 5 et 7 à 11, qui comprend:

(i) 5—70% en poids d'acide thiophosphorique, de dérivés d'acide thiophosphorique ou des pyréthrines comme ingrédients actifs;

(ii) 28—92,5% en poids d'un ou plusieurs véhicules et 0—4% en poids de sel de calcium d'acide dodécylbenzènesulfonique comme agent auxiliaire; et

(iii) 1—7,5% en poids dudit surfactant.

13. Procédé de préparation d'un surfactant de formule (I) ou (II) comme défini dans la revendication 1, qui comprend:

a) pour la préparation de composés de formule générale I, la mise en réaction d'huile de soja époxydée avec un hémiester d'un acide carboxylique formé avec un polyalkylèneglycol ayant un poids moléculaire compris entre 200 et 3000 en présence ou en l'absence de solvant, à une température de 150—200°C ce qui fournit un composé de formule (II); ou

b) pour la préparation de composés de formule générale II, la mise en réaction d'un composé de formule générale I (dans laquelle $R^1$, $R^2$, Q, A, n p et q sont comme indiqués dans la revendication 1) avec de l'ammoniac ou un composé amino primaire ou secondaire, en présence ou en l'absence de solvant.

14. Procédé selon la procédé a) de la revendication 13, qui comprend d'utiliser l'acide maléique, l'acide phtalique, l'acide adipique ou l'acide succinique comme acide dicarboxylique.

15. Surfactants de formule générale I comme défini dans la revendication 1.

16. Surfactants de formule générale II comme défini dans la revendication 1.